# FASCICULE DE BREVET EUROPEEN

(11) **EP 1 034 779 B1**
(45) Date de publication et mention de la délivrance du brevet: **27.06.2001**
(21) Numéro de dépôt: 99403302.5
(22) Date de dépôt: 28.12.1999
(51) Int. Cl.: A61K 7/48

(54) **Composition cosmétique et/ou dermatologique fluide sous forme d'émulsion eau-dans-huile.**
Wasser-in-Öl emulgierte kosmetische oder dermatologische flüssige Zusammnensetzung
Water-in-oil emulsified cosmetic or dermatological liquid composition

(30) Priorité: 08.02.1999 FR 9901446
(43) Date de publication de la demande: 13.09.2000
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 516 547
- WO-A-96/14047

## Description

La présente invention se rapporte à une composition cosmétique et/ou dermatologique sous forme d'une émulsion eau-dans-huile fluide comprenant un émulsionnant siliconé, au moins une huile hydrocarbonée à chaîne ramifiée, au moins une huile de silicone volatile et au moins un ester alkylé de polyol, et à ses utilisations pour le soin, le maquillage, le nettoyage et/ou le démaquillage de la peau, des muqueuses, des yeux et/ou des cheveux, ainsi que pour le traitement des peaux sèches.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Toutefois, ces émulsions en raison de leur texture "lourde" manquent de fraîcheur et de fluidité et sont difficiles à travailler. Elles laissent sur la peau un film gras et collant. En outre, si on augmente la quantité de phase interne (aqueuse) de manière à apporter plus de fraîcheur et un toucher moins gras, il en résulte l'obtention de crèmes souvent compactes et difficiles à étaler.

Aussi, il subsiste le besoin d'une composition sous forme d'émulsion E/H qui soit suffisamment fluide pour avoir un toucher frais, agréable à l'application et facile à étaler, tout en gardant les propriétés émollientes des émulsions à phase huileuse externe.

Ainsi, la demanderesse a découvert de façon surprenante qu'une composition sous forme d'une émulsion eau-dans-huile contenant l'association d'une huile hydrocarbonée à chaîne ramifiée et d'une huile de silicone volatile en une quantité représentant au moins 50 % de la phase huileuse, et contenant en outre un ester alkylé de polyol permettait d'obtenir une composition fluide, très fine et stable, et légère à l'application tout en conservant les propriétés habituelles des émulsions E/H.

Aussi, la présente invention a pour objet une composition cosmétique et/ou dermatologique comprenant une phase aqueuse dispersée dans une phase huileuse, caractérisée en ce qu'elle contient (1) au moins un émulsionnant siliconé, (2) au moins une huile hydrocarbonée à chaîne ramifiée, (3) au moins une huile de silicone volatile, la quantité totale d'huile hydrocarbonée à chaîne ramifiée et d'huile de silicone volatile représentant au moins 50 % en poids de la phase huileuse, et (4) au moins un ester alkylé de polyol.

La composition selon la présente invention est fluide et présente l'avantage d'être légère et fraîche à l'application tout en laissant la peau douce, mate et non collante. L'addition d'un ester alkylé de polyol permet d'obtenir une composition beaucoup plus fine et plus régulière, et donc plus stable.

Comme émulsionnants siliconés pouvant entrer dans la composition selon l'invention, on peut citer les dimethicone copolyols et les alkyl diméthicone copolyols. Comme dimethicone copolyol, on peut citer par exemple le mélange de dimethicone copolyol, de cyclomethicone et d'eau (10/88/2), commercialisé par la société Dow Corning sous la dénomination DC3225C ou DC2-5225C. Selon un mode préféré de réalisation de l'invention, on utilise comme émulsionnant siliconé un alkyl diméthicone copolyol ayant un radical alkyle comportant de 10 à 22 atomes de carbone, tel que le cétyl diméthicone copolyol comme le produit commercialisé sous la dénomination Abil EM-90 par la société Goldschmidt et le mélange de dimethicone copolyol et de cyclopentasiloxane (85/15) commercialisé sous la dénomination Abil EM-97 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning, et leurs mélanges.

L'émulsionnant siliconé est de préférence utilisé dans une proportion en matière active (c'est à dire par exemple en dimethicone copolyol ou alkyl dimethicone copolyol) allant de 0,5 à 10 % et de préférence de 1 à 6 % en poids par rapport au poids total de la composition.

La phase huileuse de la composition selon l'invention contient au moins une huile hydrocarbonée à chaîne ramifiée et au moins une huile de silicone volatile.

L'huile hydrocarbonée à chaîne ramifiée comporte de 10 à 20 atomes de carbone et peut être choisie par exemple dans le groupe comprenant l'isohexadécane, l'isododécane, les isoparaffines et leurs mélanges.

L'huile de silicone volatile peut être choisie par exemple parmi les polydiméthylsiloxanes cycliques ou linéaires, et leurs mélanges. Les polydiméthylsiloxanes cycliques ou cyclométhicones comportent d'environ 3 à 9 atomes de carbone, et de préférence de 4 à 6 atomes de carbone et peuvent être par exemple le cyclohexadiméthylsiloxane et le cyclopentadiméthylsiloxane. Les polydiméthylsiloxanes linéaires volatiles comportent de préférence d'environ 3 à 9 atomes de carbone. Les polydiméthylsiloxanes linéaires volatiles ont généralement une viscosité à 25°C inférieure ou égale à 5 cSt tandis que les cyclométhicones ont généralement une viscosité à 25°C inférieure ou égale à 10 cSt.

La quantité totale d'huile(s) hydrocarbonée(s) à chaîne ramifiée et d'huile(s) de silicone volatile(s) représente au moins 50 % en poids de la phase huileuse, le rapport entre la quantité des deux types d'huiles pouvant varier dans une large mesure du moment que chaque huile est présente, la concentration de chaque type d'huile étant de préférence d'au moins 1 % en poids par rapport au poids total de la composition. De préférence, la quantité totale d'huile(s) hydrocarbonée(s) à chaîne ramifiée et d'huile(s) de silicone volatile(s) va de 60 à 100 % en poids par rapport au poids total de la phase huileuse.

La phase huileuse peut contenir en outre tous les corps gras et notamment les huiles, classiquement utilisés dans les domaines cosmétique ou dermatologique. Les autres huiles susceptibles d'être présentes dans la phase huileuse peuvent être par exemple les huiles d'origine végétale, telles que l'huile de noyaux d'abricot et le perhydrosqualène, les huiles de synthèse comme les esters gras, les huiles de silicone non volatiles et les huiles fluorées. Les autres corps gras susceptibles d'être présents dans la phase huileuse peuvent être par exemple les acides gras et les alcools gras.

La phase huileuse de l'émulsion peut représenter par exemple de 8 à 60 % en poids et mieux de 15 à 50 % en poids par rapport au poids total de la composition.
Comme ester alkylé de polyol utilisable dans la composition de l'invention, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, le mélange d'isostéarate de sorbitan et d'isostéarate polyglycérolé, tel que le produit commercialisé sous la dénomination Arlacel 1690 par la société ICI, et leurs mélanges.

La quantité d'ester(s) alkylé(s) de polyol peut aller par exemple de 0,05 à 5 % et mieux de 0,1 à 1 % en poids par rapport au poids total de la composition.

La composition de l'invention peut éventuellement contenir aussi une ou plusieurs charges. L'addition de charges permet d'obtenir des compositions ayant des propriétés cosmétiques particulièrement satisfaisantes (douceur, matité). La ou les charges peuvent être choisies par exemple dans le groupe formé par les particules de polyamide et notamment celles vendues sous la dénomination Orgasol par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de Polytrap ; les poudres expansées telles que les microsphères creuses et notamment, les microsphères commercialisées sous la dénomination commerciale Expancel par la société Kemanord Plast ou sous la dénomination commerciale Micropearl F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination Dry-Flo par la société National Starch ; les microbilles de résine de silicone telles que celles commercialisées sous la dénomination Tospearl par la société Toshiba Silicone ; et leurs mélanges.

De manière préférée, la charge est choisie parmi les microsphères commercialisées sous la dénomination commerciale Expancel, qui sont des particules de terpolymère expansé de chlorure de vinylidène, d'acrylonitrile et de méthacrylate, et notamment celles vendues sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23. On peut aussi utiliser un mélange de ces différentes particules.

Les particules de terpolymère indiquées ci-dessus peuvent être sèches ou hydratées et peuvent être obtenues, par exemple, selon les procédés des brevets et demandes de brevets EP-A-056219, EP-A-348372, EP-A-486080, EP-A-320473, EP-A-112807 et US-A-3,615,972.

Lorsque la composition contient des charges, la quantité de charge(s) dans la composition selon l'invention peut aller de préférence de 0,01 % à 15 % et mieux de 0,1 à 5 % en poids par rapport au poids total de la composition.

La composition selon l'invention peut en outre contenir un ou plusieurs sels, et notamment un sel de magnésium tel que le sulfate de magnésium. La quantité de sel(s) peut aller par exemple de 0,1 à 5 % et de préférence de 0,5 à 1 % en poids par rapport au poids total de la composition.

En outre, de façon connue, les compositions de l'invention peuvent contenir des adjuvants habituels dans le domaine cosmétique ou dermatologique, tels que des actifs hydrophiles ou lipophiles, des conservateurs, des antioxydants, des parfums, des solvants, des charges, des filtres, des matières colorantes (piments ou colorants), des agents basiques (triéthanolamine) ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour la composition.

Selon un mode particulier de réalisation de l'invention, la composition est de préférence fluide, c'est-à-dire qu'elle a une viscosité allant d'environ 0,2 à 3 Pa.s (2 à 30 poises) et de préférence de 0,6 à 2 Pa.s (6 à 20 poises), cette viscosité étant mesurée à environ 25°C à l'aide du viscosimètre "Rhéomat Metler" équipé d'un mobile 2 (pour les viscosités inférieures à 0,7 Pa.s, c'est-à-dire 7 poises) ou d'un mobile 3 (pour les viscosités supérieures à 0,7 Pa.s).

La composition cosmétique ou dermatologique selon l'invention contient avantageusement un milieu physiologiquement acceptable, c'est-à-dire compatible avec la peau, les muqueuses, le cuir chevelu, les yeux et/ou les cheveux. Elle peut être notamment utilisée pour le soin, le maquillage (avec ajout de pigments), le démaquillage et/ou le nettoyage de la peau, des muqueuses, des yeux et/ou des cheveux.

Aussi l'invention se rapporte encore à l'utilisation cosmétique de la composition telle que définie ci-dessus pour le soin, le maquillage, le démaquillage et/ou le nettoyage de la peau, des muqueuses et/ou les cheveux.

Du fait qu'elle présente une phase huileuse externe, la composition selon l'invention peut aussi de manière avantageuse constituer une composition de soin pour les peaux sèches.

L'invention a encore pour objet l'utilisation de la composition telle que définie ci-dessus pour la préparation d'une composition destinée au traitement de la peau sèche.

La composition selon l'invention a aussi l'avantage de permettre le traitement et/ou le nettoyage de la peau sans l'agresser, ces compositions étant particulièrement bien adaptées au nettoyage des peaux, notamment des peaux sèches et sensibles.

L'invention se rapporte également à un procédé cosmétique de traitement et/ou de nettoyage de la peau, qui consiste à appliquer sur la peau, une composition telle que définie ci-dessus.

D'autres caractéristiques, aspects et avantages de la composition selon l'invention apparaîtront dans les exemples qui suivent, qui sont donnés à titre purement illustratif et nullement limitatif. Les proportions pondérales sont données en pourcentage en poids par rapport au poids total de la composition.

### Exemple 1 : Fluide adoucissant

*Phase huileuse :*
- Cétyl dimethicone copolyol 2 %
- Isohexadécane 15 %
- Polyglycéryl-4 isostéarate 0,5 %
- Cyclohexamethicone 10 %

*Charge :*
- Expancel 551 1 %

*Phase aqueuse :*
- Glycérine 5 %
- Sulfate de magnésium 0,5 %
- Conservateurs 0,4 %
- Eau qsp 100 %

Mode opératoire : on disperse avec l'aide d'une spatule la charge dans la phase huileuse, puis on disperse très doucement sous agitation vigoureuse la phase aqueuse dans le mélange obtenu.

La viscosité de ce lait, mesurée à environ 25°C à l'aide du viscosimètre "Rhéomat Metler" équipé d'un mobile 2, est égale à environ 7 poises (0,7 Pa.s).

Le lait obtenu est très doux à l'application, riche et frais à la fois, non gras, et il pénètre rapidement dans la peau qu'il laisse douce, mate et souple.

### Exemple 2 : Fluide de soin pour peaux sèches

*Phase huileuse :*
- Cétyl dimethicone copolyol 1,5 %
- Pôlyglycéryl-4 isostéarate 0,5 %
- Isohexadécane 5 %
- Cyclohexamethicone 10 %
- Perhydrosqualène 10,5 %

*Charge :*
- Expancel 551 0,5 %
- Amidon réticulé (Dry Flo) 2 %

*Phase aqueuse :*
- Glycérine 5 %
- Sulfate de magnésium 0,5 %
- Conservateurs 0,4 %
- Eau qsp 100 %

Mode opératoire : on disperse avec l'aide d'une spatule la charge dans la phase huileuse, puis on disperse très doucement sous agitation vigoureuse la phase aqueuse dans le mélange obtenu.

On obtient un lait dont la viscosité mesurée à environ 25°C à l'aide du viscosimètre "Rhéomat Metler" équipé d'un mobile 3 est d'environ 16 poises (1,6 Pa.s).

Ce lait épais est onctueux, doux et riche à l'application. Il pénètre facilement en apportant immédiatement un effet nutritif apaisant.

### Exemple 3 : Fond de teint fluide adoucissant

*Phase huileuse :*
- Cétyl dimethicone copolyol 2 %
- Polyglycéryl-4 isostéarate 0,5 %
- Isohexadécane 15 %
- Cyclohexamethicone 10 %

*Charge :*
- Pigments bruns, rouges et jaunes 5 %

*Phase aqueuse :*
- Glycérine 5 %
- Sulfate de magnésium 0,5 %
- Conservateurs 0,4 %
- Eau qsp 100 %

Mode opératoire : on disperse avec l'aide d'une spatule la charge dans la phase huileuse, puis on disperse très doucement sous agitation vigoureuse la phase aqueuse dans le mélange obtenu.

On obtient ainsi un fond de teint fluide très doux à l'application, facile à étaler. Il donne un résultat de maquillage homogène, mat et naturel. Sa viscosité mesurée à environ 25°C à l'aide du viscosimètre "Rhéomat Metler" équipé d'un mobile 3 est d'environ 10 poises (1 Pa.s).

## Revendications

1. Composition cosmétique et/ou dermatologique comprenant une phase aqueuse dispersée dans une phase huileuse, caractérisée en ce qu'elle contient (1) au moins un émulsionnant siliconé, (2) au moins une huile hydrocarbonée à chaîne ramifiée, (3) au moins une huile de silicone volatile, la quantité totale d'huile hydrocarbonée à chaîne ramifiée et d'huile de silicone volatile représentant au moins 50 % en poids de la phase huileuse, et (4) au moins un ester alkylé de polyol.

2. Composition selon la revendication 1, caractérisée en ce que l'émulsionnant siliconé est choisi parmi les alkyl diméthicone copolyols comportant un radical alkyle ayant de 10 à 22 atomes de carbone.

3. Composition selon la revendication 1 ou 2, caractérisée en ce que l'émulsionnant siliconé est choisi parmi le cétyl diméthicone copolyol, le lauryl diméthicone copolyol et leurs mélanges.

4. Composition selon l'une quelconque des revendications précédentes, caractérisée en ce que l'émulsionnant siliconé représente, en matière active, de 0,5 à 10 % en poids par rapport au poids total de la composition.

5. Composition selon l'une quelconque des revendications précédentes, caractérisée en ce que l'huile hydrocarbonée à chaîne ramifiée est choisie dans le groupe comprenant l'isohexadécane, l'isododécane, les isoparaffines et leurs mélanges.

6. Composition selon l'une quelconque des revendications précédentes, caractérisée en ce que la quantité totale d'huile(s) hydrocarbonée(s) et d'huile(s) de silicone volatile(s) va de 60 à 100 % en poids-de la phase huileuse.

7. Composition selon l'une quelconque des revendications précédentes, caractérisée en ce que la phase huileuse représente de 8 à 60 % en poids par rapport au poids total de la composition.

8. Composition selon l'une quelconque des revendications précédentes, caractérisée en ce que la quantité d'ester(s) alkylé(s) de polyol va de 0,05 à 5 % en poids par rapport au poids total de la composition.

9. Composition selon l'une quelconque des revendications précédentes, caractérisée en ce qu'elle contient en outre au moins une charge.

10. Composition selon la revendication précédente, caractérisée en ce que la quantité de charge(s) va de 0,01 % à 15 % en poids par rapport au poids total de la composition.

11. Composition selon l'une quelconque des revendications précédentes, caractérisée en ce qu'elle a une viscosité mesurée à environ 25°C à l'aide du viscosimètre "Rhéomat Metler" équipé d'un mobile 2 ou 3, allant de 0,2 à 3 Pa.s.

12. Utilisation cosmétique de la composition selon l'une quelconque des revendications précédentes pour le soin, le maquillage, le démaquillage et/ou le nettoyage de la peau, des muqueuses et/ou les cheveux.

13. Utilisation de la composition selon l'une quelconque des revendications 1 à 11, pour la préparation d'une composition destinée au traitement de la peau sèche.

14. Procédé cosmétique de traitement et/ou de nettoyage de la peau, caractérisé en ce que l'on applique sur la peau, une composition selon l'une quelconque des revendications 1 à 11.

## Patentansprüche

1. Kosmetische und/oder dermatologische Zusammensetzung, die eine in einer Ölphase dispergierte wäßrige Phase enthält, dadurch gekennzeichnet, daß sie (1) mindestens einen Siliconemulgator, (2) mindestens ein Kohlenwasserstofföl mit verzweigter Kette, (3) mindestens ein flüchtiges Siliconöl, wobei die Gesamtmenge des Kohlenwasserstofföls mit verzweigter Kette und des flüchtigen Siliconöls mindestens 50 Gew.-% der Ölphase ausmacht, und (4) mindestens einen Polyolalkylester enthält.

2. Zusammensetzung nach Anspruch 1, dadurch gekennzeichnet, daß der Siliconemulgator unter den Alkyldimethiconcopolyolen ausgewählt ist, deren Alkylgruppe 10 bis 22 Kohlenstoffatome aufweist.

3. Zusammensetzung nach Anspruch 1 oder 2, dadurch gekennzeichnet, daß der Siliconemulgator unter Cetyldimethiconcopolyol, Lauryldimethiconcopolyol und deren Gemischen ausgewählt ist.

4. Zusammensetzung nach einem der vorhergehenden Ansprüche, dadurch gekennzeichnet, daß der Siliconemulgator als Wirkstoff ausgedrückt 0,5 bis 10 Gew.-%, bezogen auf das Gesamtgewicht der Zusammensetzung, ausmacht.

5. Zusammensetzung nach einem der vorhergehenden Ansprüche, dadurch gekennzeichnet, daß das Kohlenwasserstofföl mit verzweigter Kette unter Isohexadecan, Isododecan, den Isoparaffinen und deren Gemischen ausgewählt ist.

6. Zusammensetzung nach einem der vorhergehenden Ansprüche, dadurch gekennzeichnet, daß die Gesamtmenge des Kohlenwasserstofföls oder der Kohlenwasserstofföle und des flüchtigen Siliconöls oder der flüchtigen Siliconöle im Bereich von 60 bis 100 Gew.-% der Ölphase liegt.

7. Zusammensetzung nach einem der vorhergehenden Ansprüche, dadurch gekennzeichnet, daß die Ölphase 8 bis 60 Gew.-%, bezogen auf das Gesamtgewicht der Zusammensetzung, ausmacht.

8. Zusammensetzung nach einem der vorhergehenden Ansprüche, dadurch gekennzeichnet, daß der Mengenanteil des Polyolalkylesters oder der Polyolalkylester im Bereich von 0,05 bis 5 Gew.-%, bezogen auf das Gesamtgewicht der Zusammensetzung, liegt.

9. Zusammensetzung nach einem der vorhergehenden Ansprüche, dadurch gekennzeichnet, daß sie ferner mindestens einen Füllstoff enthält.

10. Zusammensetzung nach dem vorhergehenden Anspruch, dadurch gekennzeichnet, daß der Mengenanteil des Füllstoffes oder der Füllstoffe im Bereich von 0,01 bis 15 Gew.-%, bezogen auf das Gesamtgewicht der Zusammensetzung, liegt.

11. Zusammensetzung nach einem der vorhergehenden Ansprüche, dadurch gekennzeichnet, daß sie eine bei etwa 25 °C mit einem mit einem beweglichen Teil 2 oder 3 ausgestatteten Viskosimeter "Rheomat Metler" bestimmte Viskosität von 0,2 bis 3 Pa·s aufweist.

12. Kosmetische Verwendung der Zusammensetzung nach einem der vorhergehenden Ansprüche zur Pflege, zum Schminken, zum Abschminken und/oder zur Reinigung der Haut, der Schleimhäute und/oder der Haare.

13. Verwendung der Zusammensetzung nach einem der Ansprüche 1 bis 11 zur Herstellung einer Zusammensetzung, die zur Behandlung von trockener Haut vorgesehen ist.

14. Kosmetisches Verfahren zur Behandlung und/oder zur Reinigung der Haut, dadurch gekennzeichnet, daß auf die Haut eine Zusammensetzung nach einem der Ansprüche 1 bis 11 aufgetragen wird.

## Claims

1. Cosmetic and/or dermatological composition comprising an aqueous phase dispersed in an oily phase, characterized in that it comprises (1) at least one silicone emulsifier, (2) at least one branched-chain hydrocarbonaceous oil, (3) at least one volatile silicone oil, the total amount of branched-chain hydrocarbonaceous oil and of volatile silicone oil representing at least 50% by weight of the oily phase, and (4) at least one polyol alkyl ester.

2. Composition according to Claim 1,
characterized in that the silicone emulsifier is chosen from alkyl dimethicone copolyols comprising an alkyl radical having from 10 to 22 carbon atoms.

3. Composition according to Claim 1 or 2,
characterized in that the silicone emulsifier is chosen from cetyl dimethicone copolyol, lauryl dimethicone copolyol and their mixtures.

4. Composition according to any one of the preceding claims, characterized in that the silicone emulsifier represents, as active material, from 0.5 to 10% by weight with respect to the total weight of the composition.

5. Composition according to any one of the preceding claims, characterized in that the branched-chain hydrocarbonaceous oil is chosen from the group consisting of isohexadecane, isododecane, isoparaffins and their mixtures.

6. Composition according to any one of the preceding claims, characterized in that the total amount of branched-chain hydrocarbonaceous oil (s) and of volatile silicone oil(s) ranges from 60 to 100% by weight of the oily phase.

7. Composition according to any one of the preceding claims, characterized in that the oily phase represents from 8 to 60% by weight with respect to the total weight of the composition.

8. Composition according to any one of the preceding claims, characterized in that the amount of polyol alkyl ester(s) ranges from 0.05 to 5% by weight with respect to the total weight of the composition.

9. Composition according to any one of the preceding claims, characterized in that it additionally comprises at least one filler.

10. Composition according to the preceding claim, characterized in that the amount of filler(s) ranges from 0.01% to 15% by weight with respect to the total weight of the composition.

11. Composition according to any one of the preceding claims, characterized in that it has a viscosity, measured at approximately 25°C using a "Rheomat Metler" viscometer equipped with a 2 or 3 rotor, ranging from 0.2 to 3 Pa·s.

12. Cosmetic use of the composition according to any one of the preceding claims in caring for, making up, removing make-up from and/or cleansing the skin, mucous membranes and/or hair.

13. Use of the composition according to any one of Claims 1 to 11 in the preparation of a composition intended for the treatment of dry skin.

14. Cosmetic process for treating and/or cleansing the skin, characterized in that a composition according to any one of Claims 1 to 11 is applied to the skin.
